Europäisches Patentamt

European Patent Office

Office européen des brevets

(11) Publication number: **0 187 681 B1**

## EUROPEAN PATENT SPECIFICATION

(45) Date of publication of patent specification: **20.05.92**

(51) Int. Cl.5: **C12P 13/02**, C12N 13/00, //C12N9/78

(21) Application number: **86100306.9**

(22) Date of filing: **11.01.86**

(54) Process for producing amides by use of microorganisms.

(30) Priority: **11.01.85 JP 2725/85**

(43) Date of publication of application:
**16.07.86 Bulletin  86/29**

(45) Publication of the grant of the patent:
**20.05.92 Bulletin  92/21**

(84) Designated Contracting States:
**BE DE FR GB IT NL**

(56) References cited:
**EP-A- 0 133 927**
**GB-A- 2 018 240**
**US-A- 3 880 717**

**CHEMICAL ABSTRACTS, vol. 103, no. 1, July 1985, page 465, abstract no. 5046z, Columbus, Ohio, US; & JP-A-59 213 387 (NIPPON CARBIDE INDUSTRIES CO., INC.) 03-12-1984**

(73) Proprietor: **NITTO KAGAKU KOGYO KABUSHIKI KAISHA**
**5-1, Marunouchi 1-chome**
**Chiyoda-Ku Tokyo-To(JP)**

Proprietor: **MITSUBISHI RAYON KABUSHIKI KAISHA**
**3-19, Kyobashi 2-Chome Chuo-ku**
**Tokyo-To(JP)**

(72) Inventor: **Enomoto, Kanehiko**
**36-5-106, Kyodo 5-Chome Setagaya-Ku**
**Tokyo-To(JP)**
Inventor: **Sato, Yoshiaki**
**138-21, Setogaya-Cho Hodogaya-Ku**
**Yokohama-Shi Kanagawa-Ken(JP)**
Inventor: **Nakashima, Yasutaka**
**2-6-206, Kurokawa 3-Chome**
**Otake-Shi Hiroshima-Ken(JP)**
Inventor: **Fujiwara, Atsushi**
**245-1, Kamihoshikawa Hodogaya-Ku**
**Yokohama-Shi Kanagawa-Ken(JP)**
Inventor: **Doi, Toshiaki**
**298-8, Setogaya-Cho Hodogaya-Ku**
**Yokohama-Shi Kanagawa-Ken(JP)**

(74) Representative: **Reichel, Wolfgang, Dipl.-Ing. et al**
**Reichel und Reichel Parkstrasse 13**
**W-6000 Frankfurt am Main 1(DE)**

EP 0 187 681 B1

**Description**

BACKGROUND OF THE INVENTION

Field of the Art

This invention relates to a process for hydrating a nitrile compound by the action of microorganisms having nitrilase activity to convert the nitrile compound into the corresponding amide compound. More particularly, this inveniton relates to the process for producing an amide compound by irradiation with light of the microorganisms with good yield, space time yield and productivity per bacterial cells (i.e. the amount of amide compounds to be produced per unit amount of bacterial cells used).

Recently there have been developed processes for carrying out chemical reactions by the use of microorganisms or enzymes obtained therefrom. In general, the reactions by means of microorganisms or enzymes are advantageous in that consumption of energy for the reactions is small because the reaction can be carried out at room temperature and atmospheric pressure and that desired products of high purity are easily obtained because the selectivity of the reaction to yield the desired products is very high. On the other hand, there is room for improvement with respect to the reaction activity, the life time of microorganisms or enzymes used as catalysts. Especially, there are problems when the velocity of the desired reaction (i.e. reaction activity) is low under its optimum conditions and especially at its optimum temperature and/or pH. In such a case, the productivity per bacterial cells used is reduced because large capacity reactors are required owing to low space yield, the reaction takes a longer time owing to a slow reaction velocity, and also the reaction activity is lowered.

Thus, it is fundamentally important to realize a high reaction activity of the microorganisms or enzymes to be used. Such reaction activity is a main economical factor of industrial production.

Prior Art

Processes for hydrating nitrile compounds to produce the corresponding amide compounds by the action of microorganisms having enzymatic activity which hydrates a nitrile compound into the corresponding amide compound (i.e. nitrilase activity) are described in Japanese Patent Publication No.17918/81 and No.38118/81 and Japanese Laid-Open Patent Application (Kokai) No. 86186/76 Specifications. Microorganisms play an important role in these reactions, and several microorganisms are disclosed in the specifications referred to above.

Problems

It has been found by the present inventors that the nitrilase activity cannot be exhibited sufficiently when a large metal reaction apparatus is used in order to carry out hydration reaction of nitrile compounds in an industrial scale by utilizing the nitrilase activity of these microorganisms. It has been uncertain whether these phenomena are caused by the microorganisms themselves or the material and structure of the reaction apparatus or other reasons. Thus it was necessary to solve these problems for industrial operation of a microbiological process for producing amide compounds.

A solution

As a solution for the above problems, the present inventors have already made an invention (Japanese Patent Application No. 125588/83), which has the feature that the microorganism to be used is allowed to accept light energy of at least about $1\times10^{-2}$ $\mu$mol of photons/g microbial cells second in carrying out the hydration reaction in a reaction vessel composed at least partly of a non-light-transmitting material.

After that, as a result of an intensive study of the relationship between the realization of nitrilase activity and the wavelength of light to be irradiated, it has been found by the inventors that the minimum of light energy whereby the activity of microbial cells is expected to be enhanced varies with the wavelength of light and that the minimum light energy can be considerably reduced from the above $1\times10^{-2}$ $\mu$mol of photons/g microbial cells·second.

## SUMMARY OF THE INVENTION

The present invention is based on the above finding.

According to the present invention, there is presented a process for hydrating a nitrile compound by the action of a microorganism having nitrilase activity to convert the nitrile compound into the corresponding amide compound, which process comprises conducting the reaction under the conditions wherein:

(a) the microorganism having nitrilase activity is positive Gram staining;

(b) the microbial cells are allowed to accept light energy of about $2 \times 10^{-3}$ to less than $1 \times 10^{-2}$ $\mu$mol of photons/g microbial cells·second by irradiation with light having wavelength of about 300 to 450 nm before termination of the hydration reaction; and

(c) the hydration reaction is carried out in a vessel composed at least partly of a material which does not permit transmission of light therethrough.

Thus, in the hydration reaction of a nitrile compound by the action of a microorganism having nitrilase activity according to the present invention, (i) the hydration reaction which does not substantially proceed in a reaction vessel composed of a non-light-transmitting material where light is substantially absent can proceed by irradiation of the microorganisms with light, and (ii) the hydration reaction which proceeds to some extent in a reaction vessel composed partly of a non-light-transmitting material where some amount of light is present can proceed substantially faster by the irradiation with light.

Such an effect of the light irradiation as is realized in this hydration reaction cannot be observed in microorganisms having no nitrilase activity as far as tested by the inventors.

It is well known that useful substances are produced according to microbiological methods including the use of enzymes. However, enhancement of the activity of microorganisms or their enzymes by irradiation with electromagnetic waves such as light is not believed to have been well known. On the contrary, it is generally believed that the irradiation with light is often harmful. Thus, it can be said that the effect of irradiation with light which is exhibited only in the above mentioned case has not been expected in view of the usual results of light on microorganisms.

## DETAILED DESCRIPTION OF THE INVENTION

Microorganisms to be used

As described above, it is known that a nitrile compound is converted to the corresponding amide compound by the action of microorganisms having nitrilase activity. The present inventors have found that the microorganisms belonging to the genuses described in the above mentioned Japanese Patent Publication Nos. 17918/81 and 38118/81 and Japanese Laid-Open Patent Application No. 86186/76 Specifications generally have improved activity by the irradiation thereof with light.

It is the common characteristics of the microorganisms belonging to these genuses that (i) the microorganisms have possitive Gram-staining property, (ii) they generally contain phospholipid, (iii) their cell membranes are thick and thus exhibit some resistance to the permeation therethrough with substances, and (iv) crushing of the membranes is not easy in order to take out intra-cellular enzymes.

The microorganisms to be used in the present invention are Gram-positive bacteria having nitrilase activity. Specific examples of such microorganisms include the bacteria belonging to the following genuses:

(a) Corynebacterium;

(b) Nocardia;

(c) Bacillus;

(d) Bacteridium;

(e) Micrococcus; and

(f) Brevibacterium.

Specific strain is belonging to these genuses include: Corynebacterium --- N-771 strain (FERM P 4445 deposited at a Japanese depository, Fermentation Research Institute, Agency of Industrial Science and Technology, 1, 3-Higashi 1-chome, Yatabe-machi, Tsukuba-gun, Ibarakiken, Japan, which depository is herein called FRI, on May 30, 1978) and N-774 strain (FERM P 4446 deposited at FRI on May 30, 1978); Nocardia genus --- N-775 strain (FERM P 4447 deposited at FRI on May 30, 1978), these strains being described in Japanese Patent Publication No. 17918/81 and No. 38118/81 Specifications; and the strains of Bacillus genus and others which are described in Japanese Laid-Open Patent Application No. 86186/1976

3

Specification and reported to have been deposited at CBS *and FRI. The bacteriological properties of these strains are described in these publlicly known specifications.

## Nitrile compounds and amide compounds

The nitrile compounds are represent by the general formula $R\text{-}(CN)_n$ and encompass broad ranges of compounds. The compounds include mononitriles when $n = 1$ and polynitriles when $n \geq 2$. The R is hydrogen or a saturated or unsaturated hydrocarbon residue which has a variety of numbers of carbon atom and also has a straight, branched or cyclic chain. The hydrocarbon residue can further contain amino group, hydroxyl group, a halogen, carboxyl group or other substituents which are compatible with the microorganism used.

Examples of preferable nitrile compounds include acetonitrile, propionitrile, n-butyronitrile, isobutyronitrile, n-valeronitrile, acrylonitrile, methacrylonitrile, benzonitrile, cyanopyridine, malononitrile, succinonitrile, fumaronitrile, chloroacetonitrile, $\beta$-hydroxypropionitrile, aminoacetonitrile and $\beta$-aminopropionitrile.

As a result of experiments with many nitrile compounds as shown in the following Examples, it has been found that the nitrilase activity is enhanced without exception by the irradiation with light. Thus, it is said that the present invention can be generally established with respect to nitrile compounds.

The resulting hydration reaction product is the corresponding amide compound wherein the CN group of the starting nitrile compound is hydrated into a $CONH_2$ group.

From the viewpoint of usefulness of the products, important at least at present will be the production of acrylamide from acrylonitrile and nicotinic acid amide from cyanopyridine.

## Hydration reaction

It is known to carry out the hydration reaction of a nitrile compound under the action of microorganisms having nitrilase activity. In the present invention, the hydration can be conducted in any desired embodiment so far as the object of the invention is not impaired. It is to be noted that no alteration in the hydration reaction itself has been observed by irradiation with light.

The method of the hydration reaction generally comprises contacting the starting nitrile compound with the bacterial cells in an aqueous medium for a predetermined period of time.

The bacterial cells can be in the form of a liquid culture; intact bacterial cells separated from the culture medium, preferably those washed with water; a dried product of the intact bacterial cells; or the intact cells or dried cells supported on or immobilized in a carrier. The preferred form of the bacterial cells are intact cells or those immobilized in a polymer gel such as crosslinked polyacrylamide gel or crosslinked polyvinyl alcohol.

The concentrations of the substrate (nitrile) and the bacterial cells in the aqueous medium can be suitably selected, and are generally in a concentration of the substrate ranging from about 0.01 to about 5% by weight and in a concentration of bacterial cells ranging from about 0.01 to about 2% by weight of dry cells. The reaction temperature, reacton pH and other reaction conditions can be determined, depending on the type of the microorganisms to be used. Normally, the reaction temperature is in the range of about 0 to about 20°C, and the pH is in the vicinity of about 7. A suitable buffer agent can be used in order to maintain the pH at a predetermined level.

Incidentally, preparation of the bacterial cells to be used in the hydration reaction is readily conducted by culturing the microorganism under suitable conditions. The bacterial cells separated from a culture medium and used for the hydration reaction are generally under non-proliferating conditions.

## Irradiation with light

### (1) Light

The irradiationn with light is conducted onto the bacterial cells which are in the stage before termination of the hydration reaction. The term "before termination of hydration reaction" means any point of time before termination of the hydration reaction, wherein the termination does not always mean the conversion of 100%. Since the irradiation with light is conducted onto the bacterial cells, the stage before termination of hydration reaction also includes the point of time before contacting the cells with a nitrile compound. Accordingly, the irradiation can be conducted before and/or after contacting bacterial cells with a nitrile compound.

*) CBS=Centraal Bureau Voor Schimmelstructures, Baarn, Netherlands

The irradiation can thus be conducted solely before the cells are contacted with a nitrile. When the bacterial cells irradiated with light are contacted with a nitrile compound in the absence of light, however, the effect of enhancing the nitrilase activity by the irradiation with light cannot be continued sufficiently, although the effect is revived by irradiation with light. Thus, in a preferred embodiment of the present invention, the bacterial cells are contacted with a nitrile compound and then irradiated with light. Specifically, the irradiation is conducted, for example, before and after contact of the bacterial cells with a nitrile compound.

The "light" to be irradiated according to the present invention has a wavelength of about 300 to about 450 nm. Specifically, such light can be obtained, for example, from a blacklight lamp and other mercury lamps in a wavelength range of 300 to 400 nm, but can hardly be obtained from a light for general irradiation in a wavelength range of 400 to 800 nm, such as a luminescent lamp or a incandescent lamp.

The irradiation with light can be carried out with any strength or amount as far as the effect of enhancing the activity is observed. More specifically, the irradiation should be conducted so that the bacterial cells receive light energy of about $2 \times 10^{-3}$ to less than $1 \times 10^{-2}$ $\mu$mol of photons/g cells$\cdot$second, preferably about $5 \times 10^{-3}$ to less than $1 \times 10^{-2}$ $\mu$mol of photons cells$\cdot$second. Incidentally, the use of immobilized cells sometimes decreases the reaction velocity by from about 1/2 to about 1/3 in comparison with the use of living cells at the same concentration. In this case, the irradiation requires light energy about 2 to 3 times as large as the above mentioned value. As to the light energy, the unit $\mu$mol of photons is the amount of light energy expressed by mol of photons x $10^{-6}$ or E (einstein) x $10^{-6}$ which corresponds to the energy of photons equivalent to the numbers of 1 mole molecules. The unit "g cells" is the weight expressed in gram of dried bacterial cells. The "second" is a period of irradiation expressed in second.

A light from a mercury lamp for room illumination and sunlight can partly be within the wavelength range according to the present invention. However, under the environment around a reaction vessel or other reaction apparatus of an ordinary industrial scale, the minimum amount of light energy according to the present invention cannot be given by room light as described above and/or scattered sunlight into a room.

(2) Irradiation

The irradiation can be conducted in any way, as far as bacterial cells before or in contact with a nitrile compound can successively or intermittently receive the required amount of light energy from a light source for irradiation.

The term "a light source for irradiation of bacterial cells" means a light source placed inside or outside of the reaction apparatus for irradiation of the bacterial cells with light, and also includes some light such as scattered sunlight and/or room light which may come into the reaction apparatus surrounding the reaction apparatus. Incidentally, when one makes the room where a reaction apparatus is installed excessively or extraordinarily bright so that significant light energy from the room light can come into the reaction apparatus, such a light source should be understood as a light source for irradiation according to the present invention.

More specifically, the irradiation with light can be conducted by, for example, (a) a light source placed at an upper space over a vessel containing bacterial cells (i.e. space over bacterial cells or over an aqueous suspension thereof), (b) irradiation through a window equipped at a top, side or bottom portion of the reaction vessel with an outside light source, (c) irradiation by means of a lamp placed within a liquor in the reaction vessel so that the vessel can also be used as a photochemical reactor, and (d) other methods. As necessary and if possible, reaction liquor is taken out of a vessel (especially a reaction vessel) containing bacterial cells, and the liquor containing the cells is then irradiated in another vessel by a method as described above. The vessel for irradiation in this case can, for example, comprise one or more transparent glass tubes.

The effect of light irradiation according to the present invention can be markedly exhibited, when a large reaction vessel of industrial scale is used wherein the incident light energy from an ordinary scattered sunlight or room light is short of the required amount of light energy. Such a large reaction vessel is normally made of a non-light-transmitting material and especially of a metal material in its substantial portions. In such a non-light-transmitting reaction vessel, the light energy obtained from ordinary illumination through a window or a cover of the vessel is very small. Thus, positive irradiation with light is indispensable. Incidentally, the term "reaction vessel" herein means a portion of reaction apparatus wherein at least major hydration reaction is carried out.

5

Experimentation

Example 1

Washed bacterial cells of N-774 strain (Corynebacterium) were prepared by aerobically culturing the strain in a medium (pH 7.2) comprising glucose 1%, peptone 0.5%, yeast extract 0.3%, malt extract 0.3%, and ferrous sulfate•7 hydrate 0.05% and washing the cells obtained with a 0.05M phosphate buffer. The cells were dispersed in a 0.05M phosphate buffer (pH 7.7) at a dark place to prepare a dispersion of the cells having a concentration of 2.0 g dried cells/liter. The resulting cell suspension was allowed to stand at 0°C at a dark place for 15 hours, hereinafter this sample being referred to as "dark standing", and then was divided into two portions. One portion thereof was allowed to stand at 0°C for 4 hours under irradiation at light energy of $8\times10^{-3}$ $\mu$mol of photons/g cells•second, using a 4 W blacklight lamp (supplied by Tokyo Shibaura Denki K.K., Japan), hereinafter this sample being referred to as "light standing". The other portion thereof was continued to stand at 0°C at a dark place.

By using each of the cell suspension, acrylamide was produced from acrylonitrile and the reaction velocity was studied in view of the amounts of acrylamide thus produced.

In the case of the light standing bacteria, a mixture of the cells 0.088 part (herein the quantity being shown by the weight of dried cells), acrylonitrile 1 part and 0.05M phosphate buffer (pH 7.7) 98.912 parts was subjected to reaction at 10°C in a 50 ml glass reactor under stirring for 20 minutes under the same irradiation condition as described above, hereinafter this reaction being referred to as "irradiated reaction". In the case of the dark standing bacteria, the above mentioned reaction was repeated except that light was not applied, hereinafter this reaction being referred to as "dark reaction". After the termination of the reaction, acrylamide contained in each of the reaction-liquor whose reaction had been terminated was subjected to quantitative analysis by gas chromatography. As a result, the amount of acrylamide (hereinafter referred to as AA) per unit bacterial cells was 20.4 $\mu$ mol AA/mg cells•minute in the light standing-irradiated reaction, and 0.31 $\mu$ mol AA/mg cells•minute in the dark standing-dark reaction, respectively. The ratio of these production performances was light standing-light irradiated reaction/dark standing-dark reaction = 66.

In the following examples, the value of 20.4 $\mu$ mol AA/mg cells•minute obtained by the light standing-irradiated reaction of Example 1 is shown by a relative value of 100U. Thus, the value obtained by the dark standing-dark reaction of Example 1 amounts to 1.52U.

Examples 2 through 13

The reaction liquor having composition shown in Table 1 was prepared by using each of the light standing and dark standing bacterial cells prepared as in Example 1. The reaction liquor was subjected to reaction as in Example 1. The corresponding amide compounds contained in the reaction-terminated liquor were subjected to quantitative analysis by gas chromatography to obtain the results shown in Table 2,

respectively.

Table 1

| Example No. | Bacteria cells (part) | Starting nitrile (part) | 0.05M phosphate buffer (part) | pH |
|---|---|---|---|---|
| 2 | 0.088 | acetonitrile (1) | 98.912 | 7.7 |
| 3 | " | methacrylonitrile (1) | 98.912 | " |
| 4 | " | n-valeronitrile (0.25) | 99.662 | " |
| 5 | " | cyanopyridine (1) | 98.912 | " |
| 6 | " | benzonitrile (0.125) | 99.787 | " |
| 7 | " | propionitrile (1) | 98.912 | " |
| 8 | " | n-butyronitrile (1) | 98.912 | " |
| 9 | " | malononitrile (1) | 98.912 | " |
| 10 | " | succinonitrile (1) | 98.912 | " |
| 11 | " | fumaronitrile (1) | 98.912 | " |
| 12 | " | chloroacetonitrile (1) | 98.912 | " |
| 13 | " | β-hydroxypropionitrile (1) | 98.912 | " |

Table 2

| Example | Starting nitrile compound | Amino compound produced | Light standing-irradiated reaction (U) | Dark standing-dark reaction (U) | Light standing-irradiated reaction/dark standing-dark reaction |
|---|---|---|---|---|---|
| 2 | acetonitrile | acetoamide | 22.1 | 0.37 | 59.7 |
| 3 | methacrylonitrile | methacrylamide | 70.1 | 0.62 | 113 |
| 4 | n-valeronitrile | n-valeramide | 15.3 | 0.23 | 66.5 |
| 5 | cyanopyridine | nicotinamide | 15.1 | 1.03 | 14.7 |
| 6 | benzonitrile | bemzamide | 19.7 | 0.13 | 82.3 |
| 7 | propionitrile | propioamide | 90.5 | 1.01 | 89.6 |
| 8 | n-butyronitrile | n-butylamide | 196.0 | 1.23 | 159 |
| 9 | malononitrile | malonamide | 101.0 | 0.91 | 111 |
| 10 | succinonitrile | succinamide | 297.0 | 2.01 | 148 |
| 11 | fumaronitrile | fumaramide | 57.6 | 0.37 | 156 |
| 12 | chloroacetonitrile | chloroacetamide | 250.0 | 1.70 | 147 |
| 13 | β-hydroxypropionitrile | β-hydroxyproploamide | 49.4 | 1.66 | 29.8 |

Examples 14 through 18

Washed bacterial cells were obtained by preparing as in Example 1 the strains of the following genuses: Bacillus (CBS-494) in Example 14, Bacteridium (CBS-496) in Example 15, Micrococcus (CBS-497) in Example 16, Brevibacterium (CBS-717) in Example 17, and Nocardia (N-775) in Example 18. The strains having CBS numbers are those deposited at and obtained from Centraal Bureau Voor Schimmelcultures (CBS) at Osterstraat 1, Baarn, Netherlands. The washed cells were treated as in Example 1 to obtain light-standing and dark-standing cells. By using the cells in the following formulation, acrylamide was produced from acrylonitrile and the reaction velocity was studied in view of the amounts of acrylamide thus produced.

In the case of the light-standing strains, a mixture of cells 0.088 part, acrylonitrile 1 part and 0.05 M phosphate buffer (pH 7.7) 98.912 parts was subjected to reaction under stirring at 10°C for 20 minutes (irradiated reaction). In the case of the dark-standing strains, the reaction was repeated except that light was not applied (dark reaction). Acrylamide contained in each of the reaction-terminated liquor was subjected to quantitative analysis by gas chromatography to obtain the results shown in Table 3.

Table 3

| Example | Genus of bacteria used | Strain | Light standing-light irradiation reaction (U) | Dark standing-dark reaction (U) | The light reaction/the dark reaction |
|---|---|---|---|---|---|
| 14 | Bacillus | CBS-494 | 41.5 | 7.8 | 5.3 |
| 15 | Bacteridium | CBS-496 | 93.7 | 15.2 | 6.2 |
| 16 | Micrococcus | CBS497 | 56.4 | 11.1 | 5.1 |
| 17 | Brevibacterium | CBS-717 | 91.6 | 3.1 | 29.5 |
| 18 | Nocardia | N-775 | 40.3 | 2.1 | 19.2 |

Examples 19 through 21 & Comp. Examples 1 and 2

Washed bacterial cells of N-774 strain were prepared by aerobically culturing the strain in a medium (pH 7.2) comprising glucose 1%, peptone 0.5%, yeast extract 0.3%, malt extract 0.3% and ferrous sulfate•7 hydrate 0.05% and by washing the cells obtained with a 0.05M phosphate buffer. The cells were dispersed in 0.05M phosphate buffer (pH 7.7) at a dark place to prepare 22.72 mg/m liter of the bacterial liquor.

One (1) ml each of the cell suspension was taken into 50 ml steel vessels, and 24 ml each of 0.05 mol phosphate buffer (pH 7.7) was added thereto. The resulting cell suspension was irradiated with a light source used in Example 1 at 0°C for 1 or 20 hours, wherein the light energy was controlled as shown in Table 4 by use of a filter having different optical areas placed at an upper portion of the vessel.

To each of the cell suspension irradiated with light was added 25 ml of a 5% acrylonitrile solution (in 0.05 mol phosphate buffer solution, pH 7.7). The mixture was subjected to reaction for 20 minutes under the same condition of light irradiation. For comparison, reactions were carried out in the same way by using the cell suspension without light irradiation or by irradiation with light energy of $1 \times 10^{-3}$ $\mu$mol of photons cells•second.

The quantity of acrylamide contained in these reaction liquors was subjected to quantitative analysis by gas chromatography to obtain a reaction velocity. The results are shown in Table 4.

Table 4

| Examples or Comparative Examples | Light energy applied ($\mu$mol of photons/g bact.body·sec.) | Reaction velocity after irradiation for 1 hour(U) | Reaction velocity after irradiation for 20 hours(U) |
|---|---|---|---|
| Examples 19 | $2\times10^{-3}$ | 28.4 | 30.4 |
| 20 | $5\times10^{-3}$ | 62.5 | 68.2 |
| 21 | $8\times10^{-3}$ | 96.7 | 104 |
| Comparative Examples 1 | no irradiation | 1.8 | 1.1 |
| 2 | $1\times10^{-3}$ | 5.4 | 5.8 |

**Claims**

1. A process for hydrating a nitrile compound by the action of a microorganism having nitrilase activity to convert the nitrile compound into the corresponding amide compound, which comprises conducting the reaction under the conditions wherein:
    (a) the microorganism having nitrilase activity is positive Gram staining,
    (b) the microbial cells are allowed to accept light energy of about $2 \times 10^{-3}$ to less than $1 \times 10^{-2}$ $\mu$mol of photons/g microbial cells•second by irradiation with light having wavelength of about 300 to about 450 nm before termination of the hydration reaction, and
    (c) the hydration reaction is carried out in a vessel composed at least partly of a material which does not permit transmission therethrough of light.

2. The process according to claim 1, in which the light energy is about $5 \times 10^{-3}$ to less than $1 \times 10^{-2}$ $\mu$mol of photons/g microbial cells•second.

3. The process according to any of Claims 1 to 2, in which the irradiation with light is carried out before and/or after contacting the microorganism with the nitrile compound.

4. The process according to any of Claims 1 to 3, in which the microorganism belongs to the genus selected from the group consisting of Corynebacterium, Nocardia, Bacillus, Bacteridium, Micrococcus, and Brevibacterium.

5. The processs according to any of Claims 1 to 4, in which the nitrile compound is selected from the group consisting of acetonitrile, propionitrile, n-butyronitrile, isobutyronitrile, n-valeronitrile, acrylonitrile, methacrylonitrile, benzonitrile, cyanopyridine, malononitrile, succinonitrile, fumaronitrile, chloroacetonitrile, $\beta$-hydroxypropionitrile, aminoacetonitrile, and $\beta$-aminopropionitrile.

**Revendications**

1. Procédé pour hydrater un composé nitrile, au moyen de l'action d'un micro-organisme possédant une activité nitrilase pour convertir le composé nitrile en le composé amide correspondant, et qui consiste à mettre en oeuvre la réaction dans les conditions dans lesquelles :
    (a) le micro-organisme présentant l'activité nitrilase fournit une coloration grampositive,
    (b) on amène les cellules microbiennes à recevoir une énergie lumineuse d'environ $2 \times 10^{-3}$ à moins de $1 \times 10^{-2}$ $\mu$mole de photons/g de cellules microbiennes•seconde par irradiation avec une lumière possédant une longueur d'onde d'environ 300 à 450 nm avant l'achèvement de la réaction d'hydratation, et
    (c) la réaction d'hydratation est effectuée dans un récipient réalisé au moins partiellement en un matériau qui ne transmet pas la lumière.

2. Procédé selon la revendication 1, selon lequel l'énergie lumineuse est comprise entre environ $5 \times 10^{-3}$ et moins de $1 \times 10^{-2}$ $\mu$mole de photons/g de cellules microbiennes•seconde.

3. Procédé selon l'une quelconque des revendications 1 et 2, selon lequel l'irradiation par une lumière est exécutée avant et/ou après la mise en contact du micro-organisme avec le composé nitrile.

4. Procédé selon l'une quelconque des revendications 1 à 3, selon lequel le micro-organisme fait partie du genre choisi dans le groupe comprenant le Corynebacterium, la Norcardia, le Bacillus, le Bacteridium, le Micrococcus et le Brevibacterium.

5. Procédé selon l'une quelconque des revendications 1 à 4, dans lequel le composé nitrile est choisi dans le groupe comprenant l'acétonitrile, le propionitrile, le n-butyronitrile, l'isobutyronitrile, le n-valéronitrile, l'acrylonitrile, le méthacrylonitrile, le benzonitrile, la cyanopyridine, le malononitrile, le succinonitrile, le fumaronitrile, le chloroacétonitrile, le $\beta$-hydroxypropionitrile, l'aminoacétonitrile et le $\beta$-aminopropionitrile.

**EP 0 187 681 B1**

**Patentansprüche**

1. Verfahren zur Hydratisierung eines Nitrils mit Hilfe eines Mikroorganismus mit Nitrilaseaktivität zur Umwandlung des Nitrils in das entsprechende Amid, wobei die Umsetzung unter folgenden Bedingungen durchgeführt wird:

(a) der Mikroorganismus mit Nitrilaseaktivität ist gram-positiv;

(b) man läßt die Mikrobenzellen Lichtenergie von etwa $2 \times 10^{-3}$ bis weniger als $1 \times 10^{-2}$ $\mu$mol Photonen/g Mikrobenzellen•Sekunde durch Bestrahlung mit Licht einer Wellenlänge von etwa 300 bis etwa 450 nm vor Beendigung der Hydratisierungsreaktion aufnehmen, und

(c) die Hydratisierungsreaktion wird in einem Gefäß durchgeführt, das mindestens teilweise aus einem Material besteht, das das Hindurchtreten von Licht nicht erlaubt.

2. Verfahren gemäß Anspruch 1, wobei die Lichtenergie $5 \times 10^{-3}$ bis weniger als $1 \times 10^{-2}$ $\mu$mol Photonen/g Mikrobenzellen•Sekunde beträgt.

3. Verfahren gemäß Anspruch 1 oder 2, wobei die Bestrahlung mit Licht vor und bzw. oder nach Inkontaktbringen des Mikroorganismus mit dem Nitril durchgeführt wird.

4. Verfahren gemäß einem der Ansprüche 1 bis 3, wobei der Mikroorganismus zu einem Genus aus der Gruppe Corynebakterium, Nocardia, Bazillus, Bakteridium, Mikrococcus und Brevibakterium gehört.

5. Verfahren gemäß einem der Ansprüche 1 bis 4, wobei das Nitril aus der Gruppe Acetonitril, Propionitril, n-Butyronitril, Isobutyronitril, n-Valeronitril, Acrylnitril, Methacrylnitril, Benzonitril, Cyanopyridin, Malononitril, Succhinonitril, Fumaronitril, Chloracetonitril, $\beta$-Hydroxypropionitril, Aminoacetonitril und $\beta$-Aminopropionitril ausgewählt ist.